# EUROPÄISCHE PATENTANMELDUNG

(11) **EP 0 121 856 A2**
(43) Veröffentlichungstag der Anmeldung: **17.10.1984**
(21) Anmeldenummer: 84103344.2
(22) Anmeldetag: 27.03.1984
(51) Int. Cl.: A61K 31/415, C07D 231/20

(54) **Verwendung von Pyrazolonderivaten bei der Bekämpfung des Wachstums von Tumorzellen und der Metastasenbildung, Arzneimittel hierfür und Verfahren zu deren Herstellung**

(30) Priorität: 08.04.1983 DE 3312581
(71) Anmelder: BAYER AG, 51368 Leverkusen (DE)
(72) Erfinder: Möller, Eike, Dr., D-5600 Wuppertal 1 (DE); Stegelmeier, Hartmut, Dr., D-4010 Hilden (DE); Klimetzek, Volker, Dr., D-5620 Velbert (DE)

(57) **Zusammenfassung**

Die vorliegende Erfindung betrifft die Verwendung bestimmter 1-substituierter Pyrazolon-(5)-Derivate bei der Therapie von Tumoren und zur Verhinderung der Metastasenbildung. Gegenstand der Erfindung sind auch antineoplastische und antimetastatische Arzneimittel, weiche durch den Gehalt an einer therapeutisch wirksamen Menge des Pyrazolonderivats gekennzeichnet sind.

## Beschreibung

Die vorliegende Erfindung betrifft die Verwendung bestimmter 1-substituierter Pyrazolon-(5)-Derivate bei der Therapie von Tumoren und zur Verhinderung der Metastasenbildung. Gegenstand der Erfindung sind auch antineoplastische und antimetastatische Arzneimittel, welche durch den Gehalt an einer therapeutisch wirksamen Menge des Pyrazolonderivats gekennzeichnet sind.

Das primäre Ziel einer Krebsbehandlung ist die Behandlung und Bekämpfung des Wachstums des primären Tumors. Gleichzeitig mit dieser Behandlung ist jedoch die Verhinderung der Metastasierung notwendig, die als die Absiedlung primärer Tumorzellen und deren nachfolgendes Eindringen in das Lymphsystem oder die Blutgefäße unter Verschleppung in andere Körperregionen definiert werden kann. Eine solche Ausbreitung kann durch Anhaften an den endothelialen Wandungen und anschließendes Durchdringen derselben, Ansiedelung sekundärer Tumoren in den perivaskulären Geweben und schließlich Verbreitung der Tumorzellen zu entfernten Stellen hin erfolgen. Obwohl über die klinischen Manifestationen des metastatischen Prozesses viel bekannt ist, weiß man nur wenig über die an der Metastase beteiligten biochemischen, immunologischen, genetischen und hormonalen Mechanismen. In diesem Sinne kann die Metastase als eine einzelne Erscheinung betrachtet werden, die auf einer Folge komplizierter Vorgänge beruht.

Wegen der Bedeutung sowohl der Behandlung des Wachstums des primären Tumors als auch der Verhütung der Metastase haben die Krebsforscher umfangreiche Untersuchungen durchgeführt, um die mit Tumorwachstum und Metastase verbundenen Wechselwirkungen aufzuklären.

Eine der biologischen Eigenschaften, die Tumorzellen zu besitzen scheinen, ist die Fähigkeit, mit Wirts-Blutplättchen in Wechselwirkung zu treten und sich an diese anzuheften und dadurch das Potential des Tumors zu verstärken, sich im Mikrogefäßsystem festzusetzen und an dem vaskulären Endothel zu haften. Als eine weitere Möglichkeit wurde vorgeschlagen, daß die Tumorzellen nach ihrem Festsetzen die Bildung sie umgebender, als Schutz wirkender Plättchen-Thromben auslösen können. Damit eine erfolgreiche Metastase stattfinden kann, müssen die metastatischen Zellen zunächst an dem vaskulären Endothel sich festsetzen und dort haften und intravaskulär bleiben, bis es in das umgebende Gewebe eindringt.

Wegen der Ähnlichkeiten.zwischen dem an dem Festsetzen und Anhaften der Tumorzellen an dem Endothel beteiligten Prozeß und der Bildung von Nicht-Tumor-Thromben haben viele Forscher geschlossen, daß Plättchen auf irgendeine Weise dabei eine Rolle spielen. Wegen dieser Beteiligung der Plättchen wurden zahlreiche Untersuchungen durchgeführt, um die Wirkung einer Therapie mittels Antikoagulantien auf die Metastase zu bestimmen. Die nachstehend zitierten Untersuchungen umfaßten die Verabreichung antikoagulierender Verbindungen, die potente Inhibitoren für eine Plättchen-Aggregierung sind. Die Ergebnisse sind bis heute widersprüchlich.

Von Heparin wurde berichtet, daß es die Metastase sowohl vermindert als auch erhöht, insbesondere in der Lunge [vgl. Cell. Biol. Intl. Rep. 2, 81-86 (1963), und Arch. Surg. 91, 625-629 (1965)7. Aspirin lieferte gemischte Ergebnisse [vgl. Eur.J.Cancer 8, 347-352 (1972), und Intl.J.Cancer 11, 704-718 (1973)7. Für Warfarin wurde gezeigt, daß es signifikante antimetastatische Wirkungen nach intravenöser Injektion von Tumorzellen und in spontan metastasierenden Tumoren hervorruft [vgl. Cancer 35, 5-14 (1975), und Cancer Res. 37, 272-277 (1971)7. Es wurde gezeigt, daß eine durch intravenöse Verabreichung von B-16ₐ-Melanom-Zellen induzierte Metastase durch Verabreichung des Antikoaguliermittels Prostacyclin verhindert werden kann [vgl. Cell Biol. 87, 649 (1980)].

Eine Übersicht über die Verwendung von Antikoagulantien zur Tumor-Therapie findet sich bei M. B. Donati et al, Malignancy and the Hemostatic System, Seiten 103 - 120, Raven Press, 1981.

Es wurde die Vermutung geäußert, daß der Einsatz der Therapie mittels Antikoagulantien zum Teil deshalb weniger als zufriedendstellend verlaufen ist, weil es den verwendeten Antikoaguliermitteln an Spezifität fehlt, und weil einige der Mittel auf den Tumorzellen selbst Wirkungen hervorrufen, die insgesamt die gewünschte Wirkung auf die Blutplättchen und damit auf die Metastase aufheben können.

Es wurde nunmehr gefunden, daß bestimmte Pyrazolon- derivate potente antimetastatische Mittel mit begleitender antineoplastischer Wirksamkeit darstellen.

In US-PS 3 147 276 wird beschrieben, daß gewisse 1-substituierte Pyrazolone-(5) antiinflammatorische Eigenschaften besitzen. Weiter ist bekannt, daß zahlreiche Pyrazolon-(5)-Derivate als Diuretika, Antihypertensiva und Antithrombotika eingesetzt werden können (DE-OS 2 319 280, DE-OS 2 554 701, DE-OS 2 363 511 und DE-OS 2 554 703).

Als mit Abstand stärkstes Antithrombotikum erwies sich das 3-Methyl-1-[2-(2-naphthyloxy)-ethyl]-2-pyrazolin-5-on ("Nafazatrom"), das in DE-AS 2'247 272 (US-PS 4 053 621) beschrieben wird. Von Nafazatrom ist auch bekannt geworden (siehe EP-A 0 062 319), daß es ausgezeichnete antimetastatische und antineoplastische Wirkung besitzt und daß es als Stimulator von endogenem Prostacyclin (PGI₂) aus dem Gefäßendothel wirken kann (Vermylen et al., Lancet I, 518, 1979, Chamone et al., Haemostasis 10, 297, 1981, McIntyre und Salzman, Thrombosis and Haemostasis, 46, Abstr. No. 45, (1981)).

Wie sich zeigte, ist die antithrombotische Wirkung von Nafazatrom außerordentlich strukturabhängig: So vermindert beispielsweise die Substitution von Methyl gegen Ethyl in der 3-Position des Nafazatroms dessen Wirkung bei der Thromboseprophylaxe auf etwa 1/30, während beim entsprechenden 3-Benzyl-, 3-Isopropyl-, 3-n-Propyl- sowie 3,4-Dimethyl-Derivat auch bei 100-facher Dosissteigerung gegenüber Nafazatrom kein antithrombotischer Effekt feststellbar ist. Es ist daher als äußerst überraschend anzusehen, daß solche antithrombotisch inaktiven Verbindungen und auch strukturell noch fernerliegende Pyrazolon- derivate dem Nafazatrom hinsichtlich der antineoplastischen und antimetastatischen Wirkqualität zumindest gleichwertig sind.

Die erfindungsgemäß einzusetzenden Pyrazolonderivate entsprechen der allgemeinen Formel

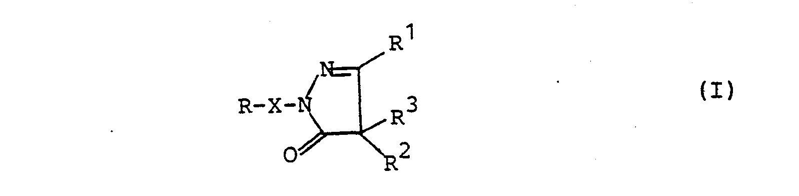

worin
R für einen Aryl- oder Heteroarylrest mit 4 bis 12 C-Atomen steht, der gegebenenfalls 1 bis 3 gleiche oder verschiedene Substituenten aus der Gruppe Halogen, Trifluormethyl, Alkyl, Alkenyl, Alkoxy, Alkylamino, Cyano, Trifluormethoxy, Nitro, Hy- _{drox}y, _{S03H}, SO n-Alkyl (n = 0 bis 2) oder SOₙ-Trifluormethyl (n = 0 bis 2) enthält, wobei unter Alkyl, Alkenyl und Alkoxy jeweils Reste mit 1 bis 4 C-Atomen zu verstehen sind,
_{R}¹ und R² gleich oder verschieden sind und Wasserstoff, NH₂ oder einen Kohlenwasserstoffrest mit 1 bis 18, vorzugsweise 1 bis 8 C-Atomen, welcher gegebenenfalls eine Hydroxy-, Ether-, Ester- oder Carboxylgruppe sowie gegebenenfalls 1 bis 3 Halogenatome enthält, darstellen,
R³ Wasserstoff oder eine Methylgruppe darstellt und
X für eine chemische Bindung oder für eine der Gruppen -O-CH₂-CH₂-, -CH₂-CH₂-, -CH2- oder 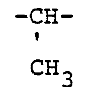 steht, wobei das Sauerstoffatom an den Rest R gebunden ist, mit der Maßgabe, daß X nicht für -O-CH₂-CH₂ steht, wenn _{R} = ß-Naphthyl; R¹ = C^{H}₃ und R² = R³ = H.

Erfindungsgemäß bevorzugt einzusetzende Verbindungen der allgemeinen Formel (I) sind solche, bei denen
R für einen gegebenenfalls substituierten Naphthyl-, Diphenyl-, Thienyl-, Phenoxyphenyl- oder Phenylrest steht, besonders bevorzugt einenα - oder B-Naphthylrest. Bevorzugt tragen diese Reste 0, 1 oder 2 Substituenten, insbesondere Br, Cl, CH₃, OCH₃ oder SO₃H.

Erfindungsgemäß werden weiterhin solche Verbindungen der allgemeinen Formel (I) bevorzugt, bei denen
_{R}¹ für eine Aminogruppe, einen aliphatischen Kohlenwasserstoffrest, vorzugsweise einen Rest mit 1 bis 4 C-Atomen welcher gegebenenfalls eine Hydroxyl-, Ether-, oder Estergruppe aufweist, besonders bevorzugt eine gegebenenfalls verzweigte C₁-C₄-Alkylgruppe steht, und
_{R}² die bevorzugte Bedeutung von R¹ (ausgenommen Aminogruppe) besitzt oder auch Wasserstoff darstellt. Besonders bevorzugt ist einer der Reste R¹ oder R² nicht Wasserstoff und _{R}¹, _{R}² und _{R}³ besitzen zusammen 2 bis 4 C-Atome.

Erfindungsgemäß bevorzugt sind schließlich auch Verbindungen, bei welchen X für eine chemische Bindung, -O-CH₂-CH₂-, -CH₂-CH₂- oder - 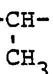 steht, insbesondere für eine chemische Bindung, -O-CH₂-CH₂- oder

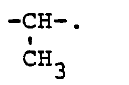

Gegenstand der Erfindung sind auch die neuen Verbindungen der allgemeinen Formel (I), bei denen weder R² noch _{R}³ Wasserstoff darstellen.

Wenn R³ = H, dann können die erfindungsgemäß einzusetzenden Verbindungen außer in der durch Formel (I) repräsentierten Form auch in einer der folgenden tautomeren Formen oder als Gemisch der möglichen tautomeren Formen vorliegen:

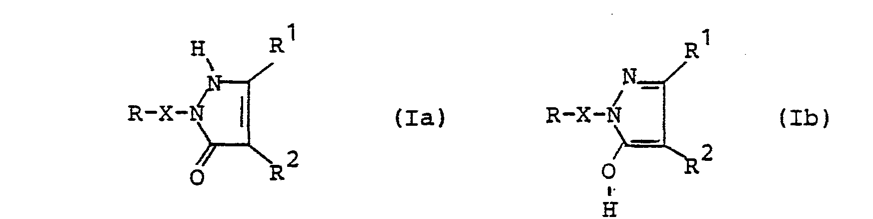

Die erfindungsgemäß einzusetzenden Pyrazolonderivate der allgemeinen Formel (I) sind aus DE-OS 2 042 663 (US-PS 3 764 321), DE-OS 2 319 280 (US-PS 3 957 814), DE-OS 2 363 511 (US-PS 4 002 641), DE-OS 2 554 701 und DE-OS 2 554 703 bekannt und können nach den dort im Detail beschriebenen Verfahren hergestellt werden.

Verbindungen der Formel (I) werden erhalten, wenn man
A) Hydrazine der Formel 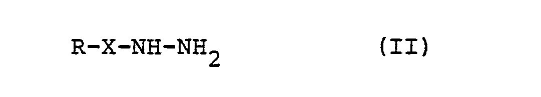 in welcher
   R und X die oben angegebene Bedeutung haben,
   mit ß-Ketosäurederivaten der Formel 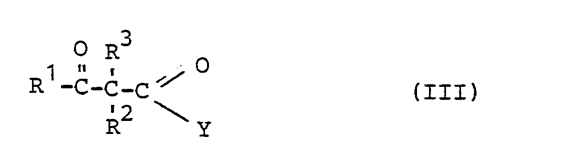 in welcher
   _{R}¹, _{R}² und _{R}³ die oben angegebene Bedeutung haben und
   Y für einen austretenden Rest, z.B. einen Hydroxy-, Alkoxy-, Aralkoxy-, Amino- oder Alkylaminorest steht,

   gegebenenfalls in Gegenwart inerte Lösungsmittel und basischer oder saurer Katalysatoren wie Alkali-und Erdalkalihydroxide und -carbonate oder wie Halogenwasserstoffsäuren, Schwefelsäure oder Sulfonsäuren, bei Temperaturen zwischen 10 und 200°C umsetzt,
   oder
3) Verbindungen der Formel 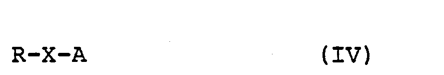 in welcher
   R und X die oben angegebene Bedeutung haben und
   A einen austretenden Rest wie Halogen oder einen Dialkyloxonium-, Dialkylsulfonium-oder Trialkylammoniumrest oder einen Aryl-oder Trifluormethylsulfonsäurerest darstellt,
   mit Pyrazolon-(5)-derivaten der Formel 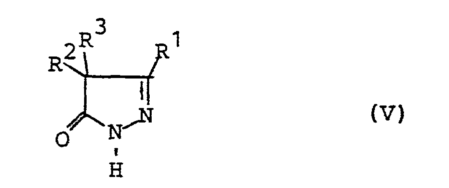 in welcher
   _{R}¹, _{R}² und _{R}³ die oben angegebene Bedeutung haben,

   gegebenenfalls in Gegenwart inerter Lösungsmittel und anorganischer oder organischer Basen wie Alkalihydroxide, -carbonate, -alkoholate, -hydride oder -amide bei Temperaturen zwischen 10 und 200°C umsetzt,
   oder (falls R² = R³ = H)
   Acetylencarbonsäurederivate der Formel 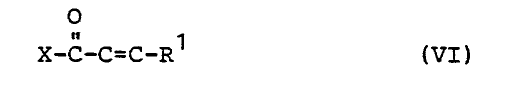 in welcher
   R¹ und Y die oben angegebene Bedeutung haben,

   mit Hydrazinen der obigen Formel (II), gegebenenfalls in Gegenwart inerter Lösungsmittel und anorganischer oder organischer Basen, bei Temperaturen zwischen 50 und 200°C umsetzt.

In den Arzneimitteln gemäß vorliegender Erfindung sind neben den erfindungsgemäß einzusetzenden Pyrazolonderivaten auch pharmazeutisch unbedenkliche Verdünnungsmittel oder Trägermaterialien enthalten. Hierunter sind nicht-toxische Substanzen zu verstehen, die nach dem Vermischen mit dem Wirkstoff diesen in einer für die Verabreichung geeigneten Form liefern. Die Bezeichnung schließt vorzugsweise Wasser und üblicherweise bei der chemischen Synthese verwendete organische Lösungsmittel mit niederem Molekulargewicht aus, außer bei Vorliegen anderer pharmazeutisch erforderlicher Bestandteile wie Salze in den richtigen Mengen, um eine isotonische Zubereitung herzustellen, Puffer, oberflächenaktive Mittel, Farb- und Geschmacksstoffe und Konservierungsmittel. Beispiele für geeignete feste und flüssige Verdünnungsmittel und Trägermaterialien sind die folgenden: Wasserhaltige Puffermittel, die durch Zusatz von Glucose oder Salzen isotonisch gemacht werden können; nicht-toxische organische Lösungsmittel wie Paraffine, pflanzliche öle, Alkohole, Glykole; gemahlene Natursteinmaterialien (beispielsweise Kaoline, Aluminiumoxide, Talkum oder Kreide); synthetische Gesteinspulver (beispielsweise hochdisperse Kieselsäure oder Silikate); und Zucker.

In der Regel enthalten die erfindungsgemäßen Arzneimittel 0,5 bis 95 Gew.-%, bevorzugt 1 bis 90 Gew.-%, besonders bevorzugt 5 bis 50 Gew.-%, an den erfindungsgemäß einzusetzenden Pyrazolonderivaten.

Die orale Verabreichung kann unter Verwendung fester und flüssiger Dosierungsformen wie Pulver, Tabletten, Dragees, Kapseln, Granulat, Suspensionen, Lösungen und dergleichen vorgenommen werden. Gegebenenfalls können die Dosierungseinheiten für die orale Verabreichung mikroverkapselt werden, um die Abgabe hinauszuzögern oder über längere Zeit hinweg zu verlangsamen, wie beispielsweise durch Überziehen oder Einbetten von teilchenförmigem Material in Polymere, Wachs oder dergleichen.

Die parenterale Verabreichung kann unter Verwendung flüssiger Dosierungsformen wie steriler Lösungen und Suspensionen erfolgen, die für die subkutane, intramuskuläre oder intravenöse Injektion bestimmt sind. Diese werden durch Suspendieren oder Auflösen einer abgemessenen Menge des Wirkstoffes in einem zur Injektion geeigneten nicht-toxischen flüssigen Streckmittel wie einem wäßrigen oder öligen Medium und Sterilisierung der Suspension oder Lösung hergestellt. Stabilisatoren, Konservierungsmittel und Emulgatoren können ebenfalls zugesetzt werden.

Im allgemeinen beträgt beim Menschen die auf das Körpergewicht bezogene Tagesdosis des Wirkstoffs für die parenterale Verabreichung 0,01 bis 50 mg/kg, vorzugsweise 0,1 bis 10 mg/kg, und für die orale Verabreichung 0,1 bis 500 mg/kg, vorzugsweise 0,5 bis 100 mg/kg, besonders bevorzugt 1 bis 50 mg/kg.

Die Dosierungseinheit (Tablette, Kapsel, etc.) enthält in der Regel 1 bis 100 mg, bevorzugt 5 bis 50 mg, besonders bevorzugt 10 bis 30 mg, an den erfindungsgemäß einzusetzenden Pyrazolonderivaten.

Zur Bestimmung der antimetastatischen und antineoplastischen Eigenschaften der erfindungsgemäß zu verwendenden Pyrazolonderivate wurde die folgende Arbeitsweise angewandt.

### A. In vivo-Erhaltung der Tumor-Linien

Es wurden dieselben Tumor-Zellinien wie in EP-A 0 062 319 verwendet und auf die gleiche Weise in vivo gehalten. ;

### B. Isolierung und Suspension der Tumorzellen

Tumorzellen wurden dann aseptisch aus den Wirtsmäusen entnommen und unter Anwendung sequenzieller Collagenase-Verdauung dispergiert, wie unten beschrieben. Die entnommenen Tumoren wurden in Würfel (4 x 4 mm) zerteilt, und das gewürfelte Gewebe wurde auf 6 bis 8 sterile Polycarbonat-Erlenmeyerkolben aufgeteilt (etwa 500 mg/Kolben). Ein Anteil von 10 ml einer "Tumordispersionslösung" (TDS) wurde in jeden Kolben hinzugefügt.

Die TDS wurde durch Vermischen der Zusammensetzung A und der Zusammensetzung B hergestellt, die nachstehend beschrieben werden.

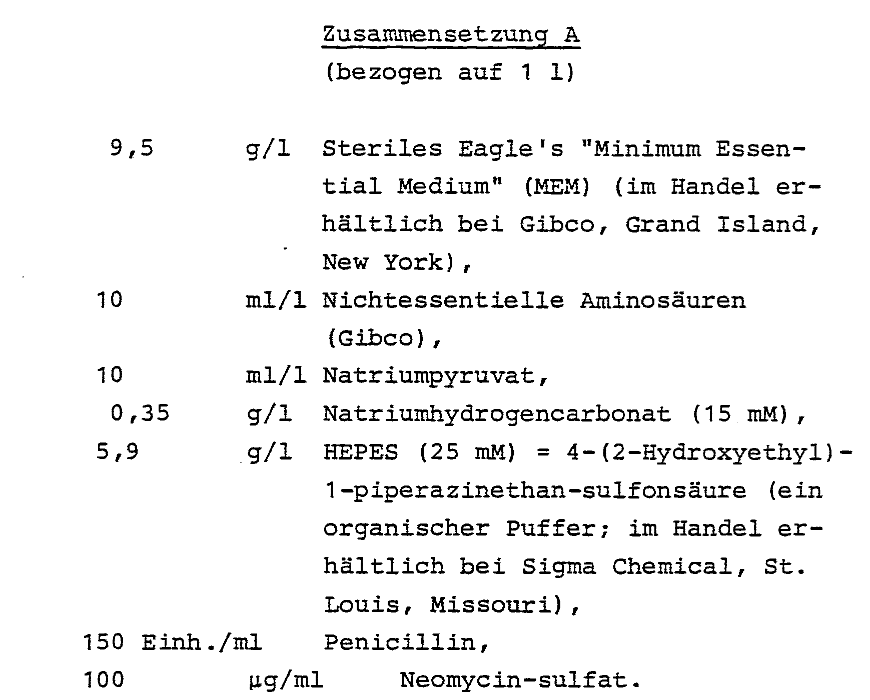

Die Antibiotika wurden zugesetzt, um sicherzustellen, daß keine bakterielle Verunreinigung stattfand.

Zusammensetzung B ist eine trockene Mischung enthaltend .Collagenase mit wenig Clostripain und anderer proteolytischer Aktivität; Desoxyribonuclease (DNase), um das von den beschädigten Zellkernen freigesetzte Desoxyribonukleoprotein aufzulösen; Lima-Bohnen- oder Sojabohnen-Trypsininhibitoren, um jegliche tryptische Restwirkung auszuschließen; menschliches Serumalbumin, um nicht-spezifische Protease-Aktivität auszuschalten und Peroxy- sowie Hydroperoxy-Fettsäuren zu absorbieren, die von beschädigten Membranen freigesetzt wurden.

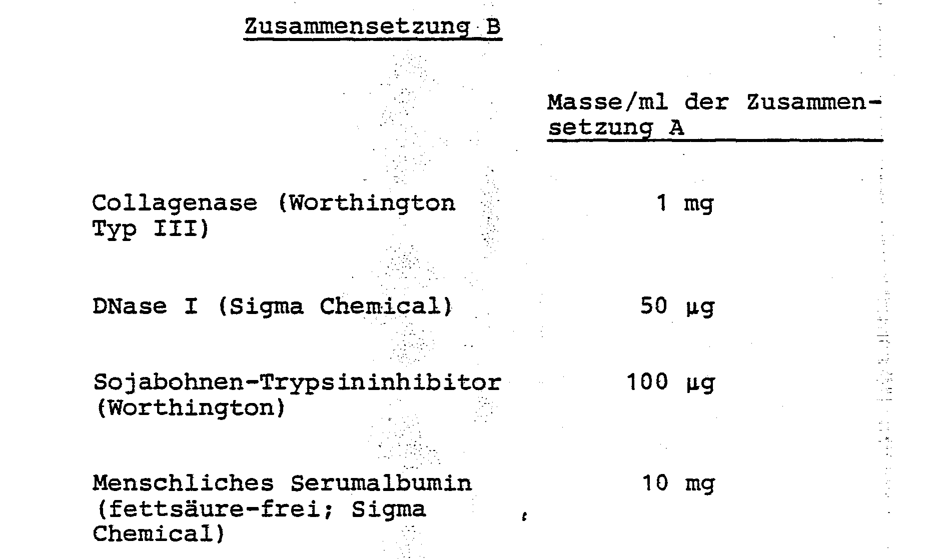

Zusammensetzung B wurde eingewogen und in einen Kolben gegeben, und sodann wurden 100 ml der Zusammensetzung A hinzugefügt.

Das gewürfelte Gewebe in der TDS wurde dann unter Luft in einem Dubnoff-Stoffwechsel-Schüttler (90 Schwingungen/min) dispergiert (30 min; 37°C). Die überstände wurden durch ein Seihtuch in sterile 50 ml-Rundboden-Zentrifugenröhrchen auf Polycarbonat gesammelt und 10 min (25°C) mit 900 U/min(100 g) in einem Sorvall SS-34 -Rotor zentrifugiert. Nach dem Zentrifugieren wurde die überstehende Fraktion verworfen. Das erhaltene zelluläre Material (Pellets) wurde zweimal mit MEM-Lösung gewaschen, wieder in MEM suspendiert und bei 4°C gehalten.

Zu dem verbleibenden gewürfelten Gewebe wurde ein Anteil von 10 ml TDS hinzugefügt, und das Gewebe wurde in einem Stoffwechselschüttler inkubiert, wie oben beschrieben, außer daß die Zeitdauer 60 min betrug. Das Zentrifugieren wurde wiederholt, und die wieder suspendierten Zellen wurden vereinigt.

Die Lebensfähigkeit der Zellen wurde nach der Vitalfarbstoff-Ausschlußmethode (vital dye exclusion method) Lvgl. Exptl. Cell Res. 13, 341-347 (1957)] bestimmt. Die Zellenzahl (cell count) wurde in einem Hämocytometer bestimmt.

### C. Wirkung der erfindungsgemäß zu verwendenden Pyrazolon- derivate auf Wachstum und Metastase von Tumorzellen

Die auf diese Weise erhaltenen B-16 -Melänomzellen wura den wie nachstehend beschrieben zur Prüfung der antimetastatischen und antineoplastischen Wirksamkeit der zu untersuchenden Verbindungen verwendet.

### (1) Metastase

Wie bereits weiter oben festgestellt wurde, ist die Metastase eine einzelne Erscheinung, die durch eine Reihe komplizierter Vorgänge verursacht wird. Gegenwärtig finden zwei "Modell"-Systeme für die Untersuchung der in vivo-Metastase verbreitete Anwendung. Das erste Modellsystem umfaßt die subkutante Injektion der Tumorzellen in das Tier. Die subkutante Injektion der Tumorzellen und anschließende Entwicklung eines Primärtumors, gefolgt von spontaner Metastase, wird als "volle" Metastase betrachtet. Ein anderes Modellsystem umfaßt die Injektion der Tumorzellen durch die Schwanzvene. In Anbetracht der Komplexität der Metastase gilt die Schwanzvenen-Injektion nur als partielles Modellsystem, da es nur die Vorgänge während des letzten Teils der Metastase wiedergibt. Das Schwanzvenen-Modellsystem gilt jedoch andererseits als außerordentlich günstig im Hinblick auf eine Standardisierung der Versuchsbedindungen Lvgl. "Methods in Cancer Research", Kapitel VII, Academic Press, Inc., 19787.

Als Kontrolltier wurden (unbehandelte) C57B1/6J-Mäuse nach folgender Arbeitsweise auf volle Metastase untersucht: Zellsuspensionen der B-16ₐ-Melanom-Zellen, die wie oben unter A und B beschrieben erhalten worden waren, wurden subkutan in die rechte Achselhöhlenregion der männlichen C57BL/6J-Mäuse injiziert (0,66 mm- = 26 gauge -Nadel; 0,2 ml). Injiziert wurden verschiedene Mengen der Zellsuspensionen im Bereich von 5 x 10⁴ bis 1 x 10 Zellen. Versuche zur partiellen Metastase wurden in der Weise durchgeführt, daß Tumorzellen über die Schwanzvene in die (unbehandelten) Kontroll-Mäuse injiziert wurden. Die Tiere wurden unter identischen Bedingungen der Temperatur, Lichteinwirkungsdauer, Ernährung etc. gehalten. Nach einem Beobachtungszeitraum von 17 bis 30 Tagen wurden die für die volle Metastase und für die partielle Metastase verwendeten Tiere getötet, und die Lungen, Leber, Nieren und Milz wurden.entnommen.

Die Zahl der metastatischen Knötchen wurde in jedem Organ bestimmt. Die Untersuchung der Kontroll-Mäuse auf metastatische Knötchen ergab bei 100 % der Tiere positive Befunde in bezug auf metastatische Lungentumoren; die Inkubationszeit für die Erzeugung solcher Metastasen betrug für die B-16 ₐ-Tumorzellen 23 bis 30 Tage. In Leber, Nieren und Milz wurden keine sichtbaren Knötchen beobachtet.

### (2) Antineoplastische Wirksamkeit

Die antineoplastische Aktivität wurde in vivo in der Welse bestimmt, daß den Mausen subkutan Tumorzeller. injiziert wurden und das Auftreten von Tumorzellenwachstum sowie das Gewicht der wachsenden Tumorzellen in unbehandelten Kontrolltieren und in mit der Pyrazolon-Verbindung behandelten Tieren bestimmt wurden.

Die Wirkung von erfindungsgemäß einzusetzenden Pyrazolonderivaten auf die durch Schwanzvenen-Injektion von Tumorzellen hevorgerufene Metastase bzw. auf die volle Metastase aufgrund der subkutanen Injektion von Tumorzellen wird in den Beispielen 1 - 4 gezeigt.

### Herstellung von 1-/2-(2-Naphthoxi)ethy173,4,4-trimethyl- pyrazolin-5-on

Eine Mischung aus 5 g (0.025 Mol) 2-(2-Naphthoxi)-ethylhydrazin und 3,6 g (0,025 Mol) 2,2-Dimethylacetessigsäuremethylester wird in 40 ml absolutem Ethanol 4 h unter Rückfluß erhitzt. Nach dem Abkühlen wird die Reaktionslösung am Rotationsverdampfer eingeengt und der Rückstand mit Ether aufgenommen. Man extrahiert zweimal mit 2n Natriumhydroxid, trocknet die organische Phase über Natriumsulfat und destilliert das Lösemittel im Vakuum ab. Der Rückstand wird aus Ether und dreimal aus Ethanol umkristallisiert.

Ausbeute: 1,9 g (26%); Fp. = 115-117°C.

### Beispiel 1

5 mg des jeweiligen Pyrazolonderivats wurden in 0,5 % Tylose aufgenommen und dann durch Behandlung mit Ultraschall (30 sec) gelöst. Die endgültige Konzentration der Substanzen wurde durch Verdünnung der Lösungen mit 0,5 % Tylose eingestellt.

Syngeneische C57BL/6J-Mäuse wurden während eines Zeitraumes von drei Tagen mit 0,005 mg/kg bis 5 mg/kg der beiden Substanzen in 0,2 ml 0,5 % Tylose peroral behandelt. Kontrolltiere erhielten täglich 0,2 ml 0,5 % Tylose. Am vierten Tag wurde den vorbehandelten Mäusen und Kontrollmäusen ein Suspension von 5 x 10⁴ B-16a Tumorzellen über die Schwanzvene injiziert. Die Kontrollmäuse und die behandelten Mäuse wurden unter identischen Bedingungen der Temperatur, Lichteinwirkungsdauer, Ernährung etc. gehalten. Die Mäuse wurden 24 Tage nach erfolgter Schwanzvenen-Injektion getötet, und ihre Lungengewebe wurden untersucht. Der Grad der Metastasierung wurde nach Zahl und Größe der makroskopisch sichtbaren Metastasen mit Punktzahlen von 1 - 10 bewertet, welche die Relation zwischen Metastasenbedeckter und - freier Organfläche ausdrücken. Dabei werden die Punktzahlen nach folgenden Rahmenkriterien vergeben: 1 = 1 - 2 gerade erkennbare Metastasen, 5 = etwa 50 % der Organoberfläche ist von Metastasen bedeckt, 10 = fast keine normale Organoberfläche sichtbar.

Wie aus den in Tabelle 1 zusammengestellten Daten zu ersehen ist, wird bei Verabreichung der Pyrazolon-Derivate die Zahl und Größe der Metastasen vermindert und zwar im Bereich von 0,005 mg-5mg/kg.

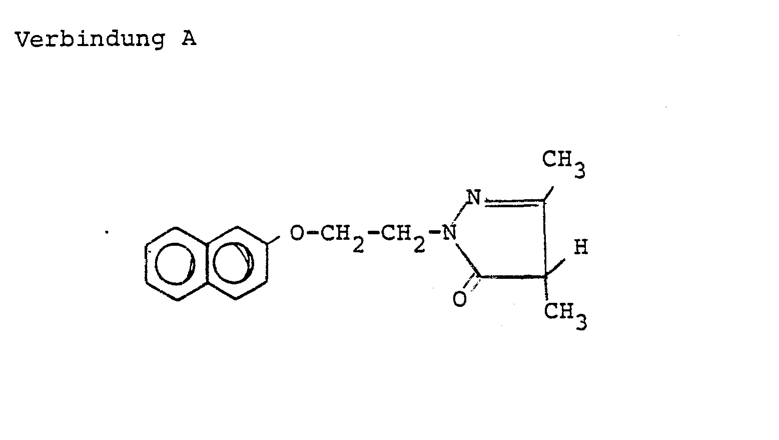

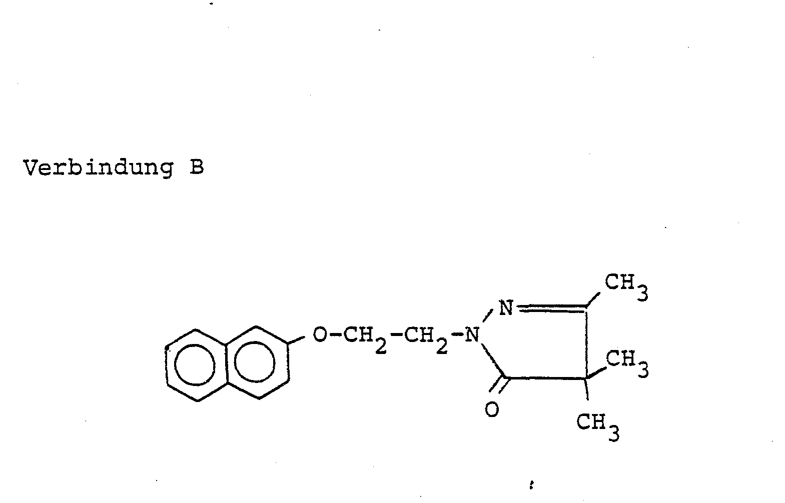

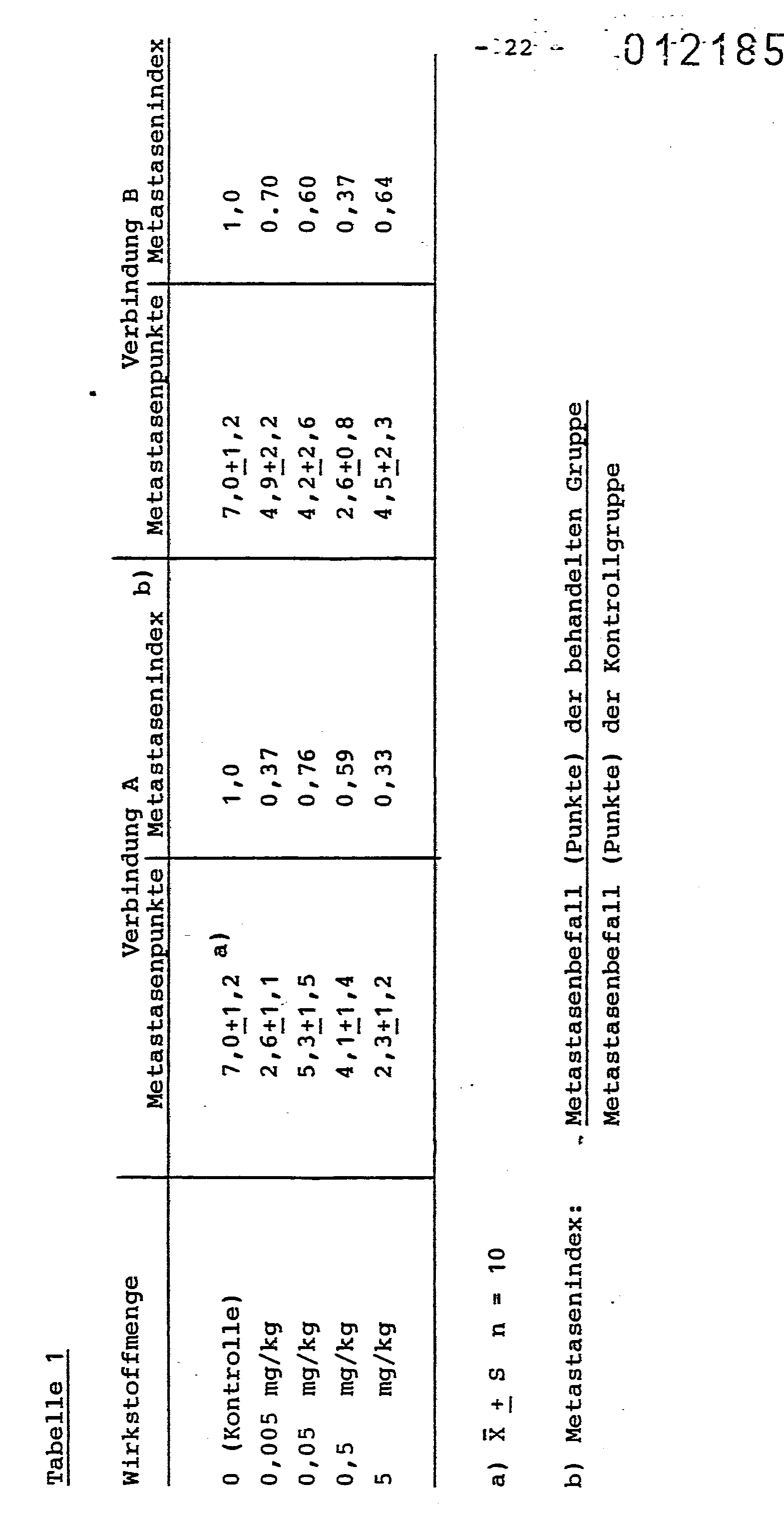

### Beispiel 2

5 mg des jeweiligen Pyrazolonderivats wurden in 0,5 % _{T}y-lose aufgenommen und dann durch Behandlung mit Ultraschall (30 sec) gelöst. Die endgültige Konzentration der Substanzen wurde durch Verdünnung der Lösungen mit 0,5 % Tylose eingestellt.

Syngeneische C57BL/6J-Mäuse wurden während eines Zeitraumes von drei Tagen vor der Tumorinjektion und 2 Tagen nach der Tumorinjektion täglich mit 0,005 mg/kg bis 5 mg/kg der beiden Substanzen in 0,2 ml 0,5 % Tylose peroral behandelt. Kontrolltiere erhielten 0,2 ml 0,5 % Tylose. Am vierten Tag wurde den. vorbehandelten Mäusen und Kontrollmäusen eine Suspension von 5 x 10⁴ B-16a Tumorzellen über die Schwanzvene injiziert.

Wie aus den in Tabelle 2 zusammengestellten Daten zu ersehen ist, wird bei Verabreichung der Pyrazolon-Derivate die zahl und Größe der Metastasen vermindert und zwar im Bereich von 0,005 mg - 5 mg/kg.

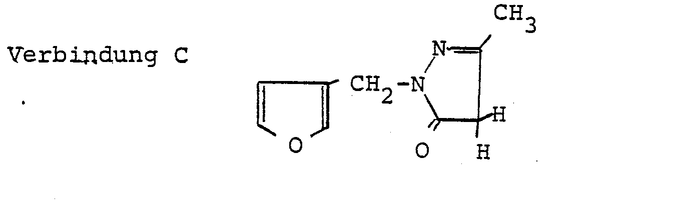

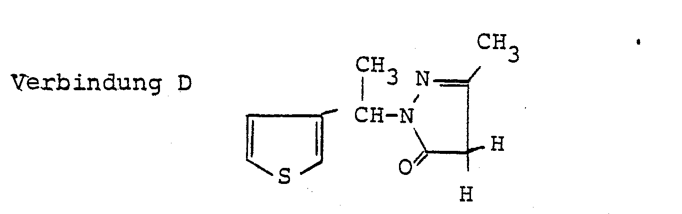

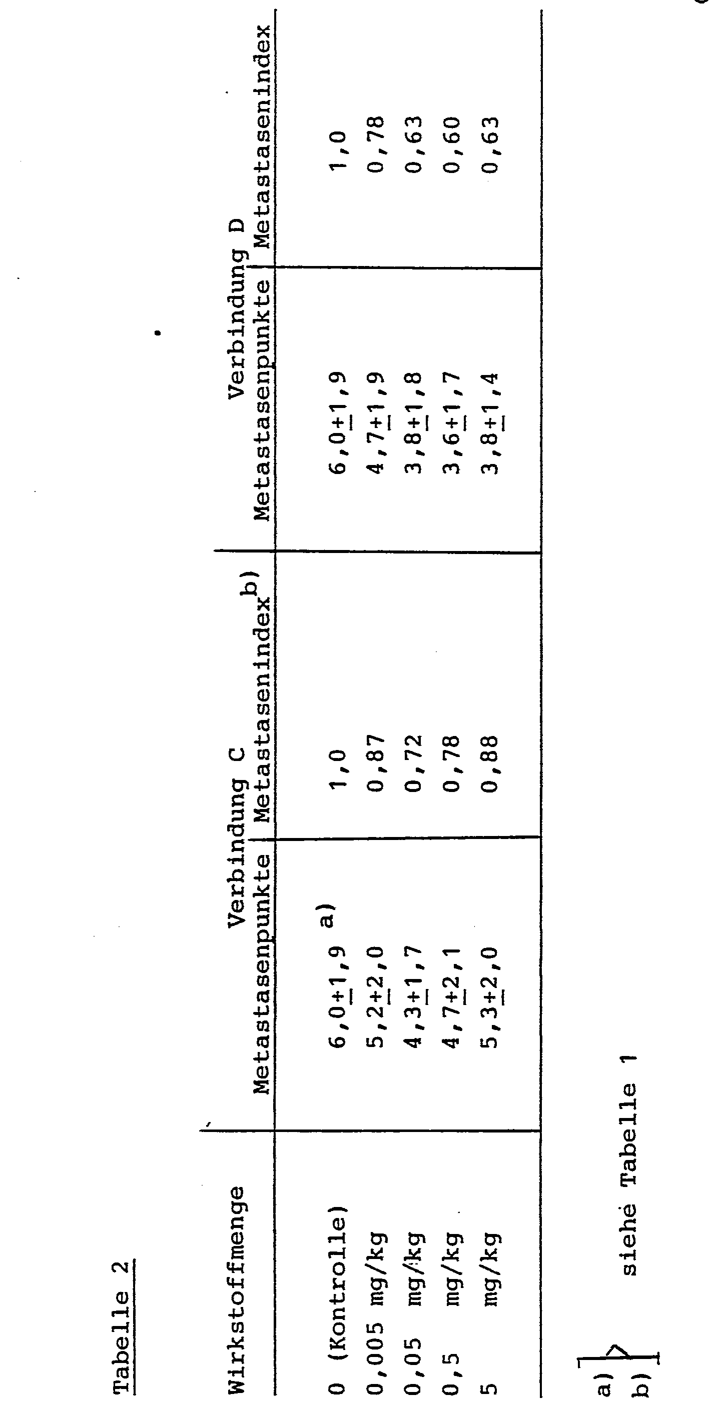

### Beispiel 3

20 mg des jeweiligen Pyrazolonderivats wurden in 0,5 % Tylose aufgenommen und dann durch Behandlung mit Ultraschall (30 sec) gelöst. Die endgültige Konzentration der Substanzen wurde durch Verdünnung der Lösungen mit 0,5 % Tylose eingestellt.

Syngeneische Tiere wurden während eines Zeitraumes von drei Tagen vor der Tumorinjektion und 2 Tagen nach der Tumorinjektion täglich mit 2 mg bis 200 mg/kg der Substanzen in 0,2 ml 0,5 % Tylose peroral behandelt. Kontrolltiere erhielten 0,2 ml 0,5 % Tylose. Am vierten Tag wurde den vorbehandelten Mäusen und Kontrolltieren eine Suspension von 5 x 10⁴ B-16a Tumorzellen über die Schwanzvene injiziert.

Wie aus den in Tabelle 3 zusammengestellten Daten zu ersehen ist, wird bei Verabreichung der Pyrazolon-Derivate die Zahl und Größe der Metastasen vermindert und zwar im Bereich von 2 mg - 200 mg/kg.

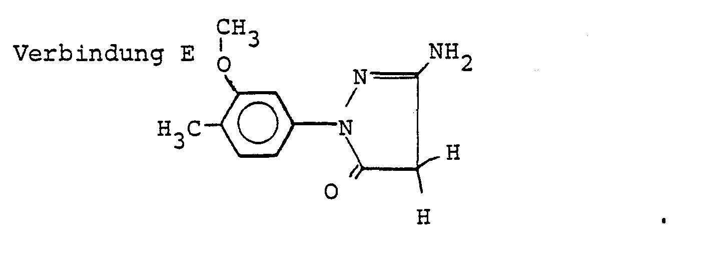

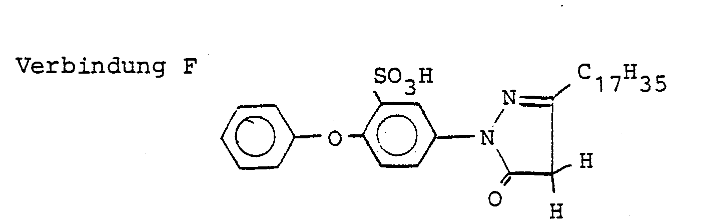

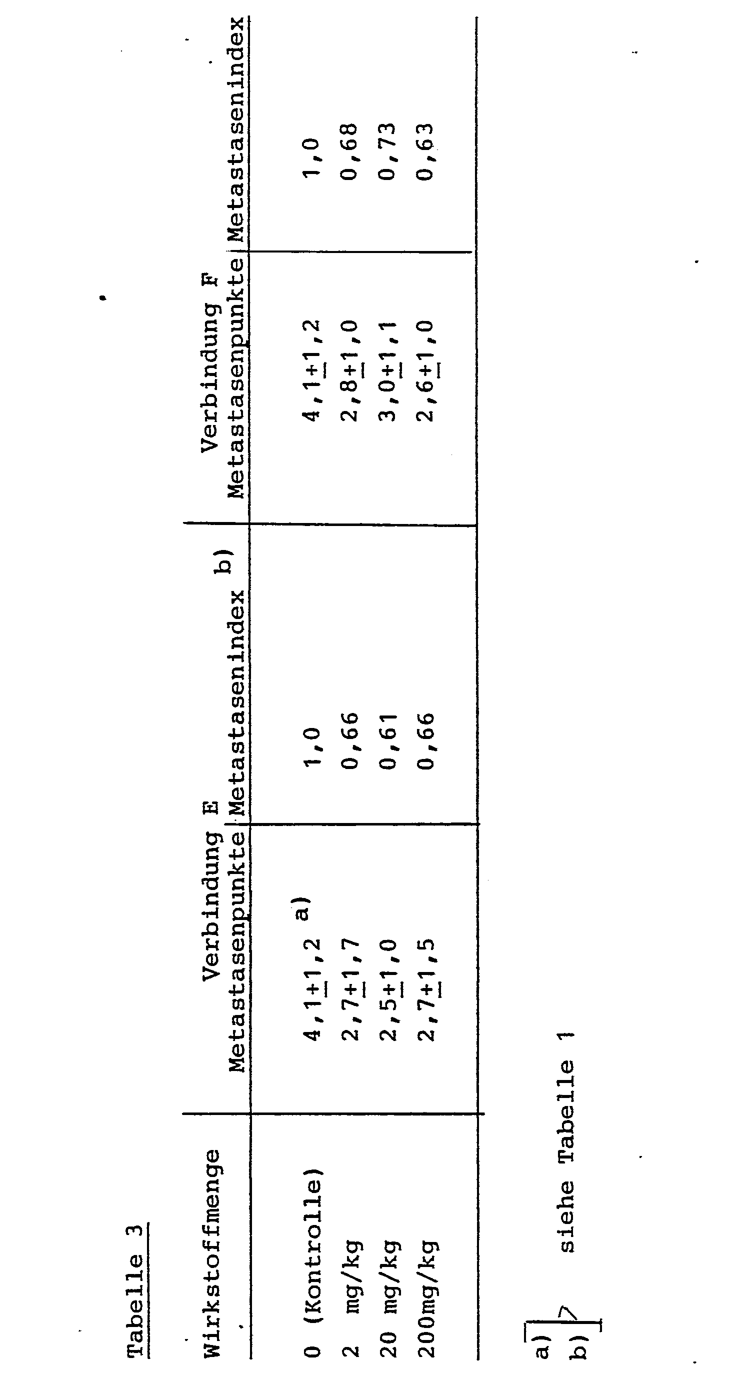

### Beispiel 4

Die Wirkung der Verabreichung der Pyrazolon-Verbindungen über einen längeren Zeitraum hinweg auf die Zahl der'metastatischen Lungen-Kolonien des B-16a Melanoms wurde wie nachstehend für Verbindung A beschrieben bestimmt.

10 mg Verbindung A wurden in 0,5 % Tylose aufgenommen und dann durch Behandlung mit Ultraschall (30 sec) gelöst. Die endgültige Konzentration der Substanz wurde durch Verdünnung der Lösung mit 0,5 % Tylose eingestellt. Die Substanz wurde jeden Tag neu gelöst.

Syngeneische C57BL/6J-Mäusen wurde eine Suspension von 1,0 x 10⁵ B-16a-Zellen subkutan injiziert, die wie vorstehend beschrieben hergestellt wurde. Vom Tage nach der Tumorzell-Injektion ab erhielten die Mäuse 27 Tage peroral eine tägliche Einzeldosis von 0 bis 100 mg/kg der Pyrazolon-Verbindung. Die Kontroll-Mäuse und die behandelten Mäuse wurden unter identischen Bedingungen der Temperatur, Lichteinwirkungsdauer, Ernährung etc. gehalten. Die Mäuse wurden nach 28 Tagen getötet und die Zahl und Größe der metastatischen Knötchen in der Lunge bestimmt.

Die bei der Untersuchung des Lungengewebes erhaltenen experimentellen Daten sind in der folgenden Tabelle 4 zusammengestellt.

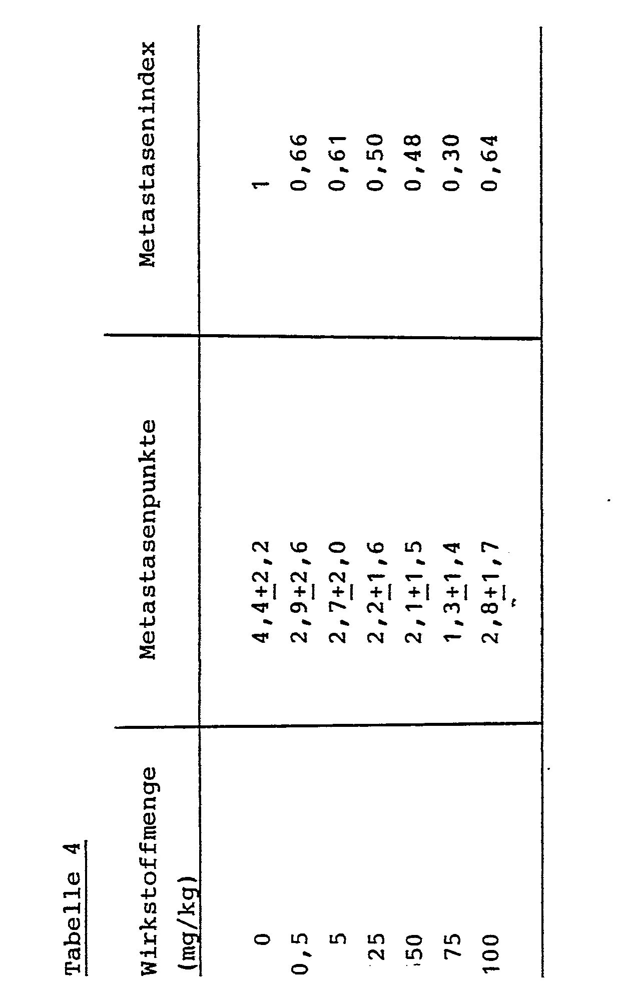

## Patentansprüche

1. Verwendung von Verbindungen der allgemeinen Formel (I) 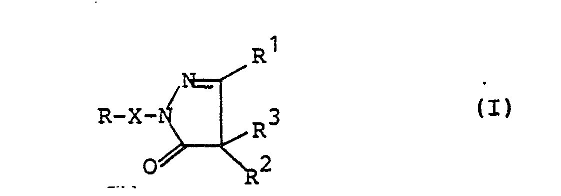 worin
R für einen Aryl- oder Heteroarylrest mit 4 bis 12 C-Atomen steht, der gegebebenfalls 1 bis 3 gleiche oder verschiedene Substituenten aus der Gruppe Halogen, Trifluormethyl, Alkyl, Alkenyl, Alkoxy, Alkylamino, Cyano, Trifluormethoxy, Nitro, Hydroxy, S0₃H, SOₙ-Alkyl (n ⁼ 0 bis 2) oder SOₙ-Trifluormethyl (n = 0 bis 2) enthält, wobei unter Alkyl, Alkenyl und Alkoxy jeweils Reste mit 1 bis 4 C-Atomen zu verstehen sind,
_{R}¹ und _{R}² gleich oder verschieden sind und Wasserstoff, NH₂ oder einen Kohlenwasserstoffrest mit 1 bis 18 C-Atomen, welcher gegebenenfalls eine Hydroxy-, Ether-, Ester- oder Carboxylgruppe sowie gegebenenfalls 1 bis 3 Halogenatome enthält, darstellen,
_{R}³ Wasserstoff oder eine Methylgruppe darstellt und
X für eine chemische Bindung oder für eine der Gruppen -O-CH₂-CH₂-, -CH₂-CH₂-, -CH₂- oder 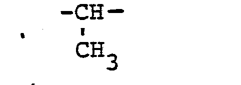 steht, wobei das Sauerstoffatom an den Rest R gebunden ist, mit der Maßgabe, daß X nicht für -O-CH₂-CH₂- steht, wenn R = B-Naphthyl; R¹ = CH₃ und R² = R³ = H; bei der Therapie von Tumoren und zur Bekämpfung der Metastasenbildung.

2. Verwendung nach Anspruch 1, dadurch gekennzeichnet, daß R für einen gegebenenfalls durch 1 oder 2 Substituenten, insbesondere Cl, Br, CN₃, OCH₃ oder S0₃H substituierten Naphthyl-, Diphenyl-, Thienyl-, Phenoxyphenyl- oder Phenylrest steht.

3. Verwendung nach Ansprüchen 1 oder 2, dadurch gekennzeichnet, daß R¹ und R² für Wasserstoff oder einen Kohlenwasserstoffrest mit 1 bis 8 C-Atomen, welcher gegebenenfalls eine Hydroxyl-, Ether- oder Estergruppe aufweist, vorzugsweise eine gegebenenfalls verzweigte C₁-C₄-Alkylgruppe, stehen, wobei entweder _{R}¹ oder R² ≠ H.

4. Verwendung nach Ansprüchen 1 bis 3, dadurch gekennzeichnet, daß R¹, R² und R³ zusammen 2 bis 4 C-Atome aufweisen.

5. Verbindung der allgemeinen Formel (I) 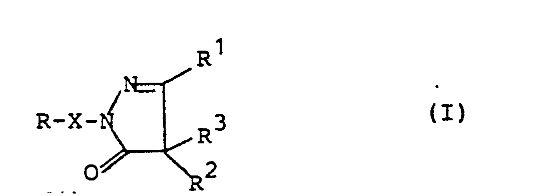 worin
R für einen Aryl- oder Heteroarylrest mit 4 bis 12 C-Atomen steht, der gegebebenfalls 1 bis 3 gleiche oder verschiedene Substituenten aus der Gruppe Halogen, Trifluormethyl, Alkyl, Alkenyl, Alkoxy, Alkylamino, Cyano, Trifluormethoxy, Nitro, Hydroxy, SO₃H, SOₙ-Alkyl (n = 0 bis 2) oder SOn-Trifluormethyl (n = 0 bis 2) enthält, wobei unter Alkyl, Alkenyl und Alkoxy jeweils Reste mit 1 bis 4 C-Atomen zu verstehen sind,
R¹ und _{R}² gleich oder verschieden sind und Wasserstoff, NH₂ oder einen Kohlenwasserstoffrest mit 1 bis 18 C-Atomen, welcher gegebenenfalls eine Hydroxy-, Ether-, Ester- oder Carboxylgruppe sowie gegebenenfalls 1 bis 3 Halogenatome enthält, darstellen, mit der Maßgabe, daß R² nicht für Wasserstoff steht,
R eine Methylgruppe darstellt und
X für eine chemische Bindung oder für eine der Gruppen -O-CH₂-CH₂-, -CH₂-CH₂-, -CH₂- oder 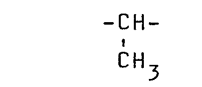 steht.

6. Verbindung nach Anspruch 5, dadurch gekennzeichnet, daß R für einen gegebenenfalls durch 1 oder 2 Substituenten, insbesondere Cl, Br, CH₃, OCH₃ oder S0₃H substituierten Naphthyl-, Diphenyl-, Thienyl-, Phenoxyphenyl- oder Phenylrest steht.

7. Verbindung nach Ansprüchen 5 oder 6, dadurch gekennzeichnet, daß R¹ und R² für Wasserstoff oder einen Kohlenwasserstoffrest mit 1 bis 8 C-Atomen, welcher gegebenenfalls eine Hydroxyl-, Ether- oder Estergruppe aufweist, vorzugsweise eine gegebenenfalls verzweigte C₁-C₄-Alkylgruppe, stehen, wobei R² ≠ H.

8. Verbindung der Formel

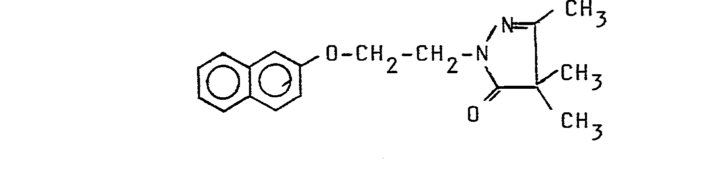

9. Verbindungen gemäß Anspruch 5 bis 8 zur Verwendung bei der Therapie von Tumoren und bei der Bekämpfung der Metastasenbildung.

10. Verfahren zur Herstellung von Verbindungen der allgemeinen Formel (I) 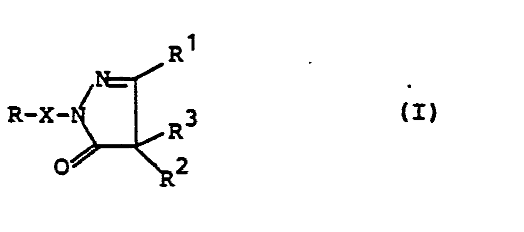 worin
R für einen Aryl- oder Heteroarylrest mit 4 bis 12 C-Atomen steht, der gegebebenfalls 1 bis 3 gleiche oder verschiedene Substituenten aus der Gruppe Halogen, Trifluormethyl, Alkyl, Alkenyl, Alkoxy, Alkylamino, Cyano, Trifluormethoxy, Nitro, Hydroxy, SO₃H, SOₙ-Alkyl (n ⁼ 0 bis 2) oder SOₙ-Trifluormethyl (n = 0 bis 2) enthält, wobei unter Alkyl, Alkenyl und Alkoxy jeweils Reste mit 1 bis 4 C-Atomen zu verstehen sind,
R¹ und _{R}² gleich oder verschieden sind und Wasserstoff, NH₂ oder einen Kohlenwasserstoffrest mit 1 bis 18 C-Atomen, welcher gegebenenfalls eine Hydroxy-, Ether-, Ester- oder Carboxylgruppe sowie gegebenenfalls 1 bis 3 Halogenatome enthält, darstellen, mit der Maßgabe, daß R² nicht Wasserstoff bedeutet,
R³ eine Methylgruppe darstellt und
X für eine chemische Bindung oder für eine der Gruppen -O-CH₂-CH₂-, -CH₂-CH₂-, -CH₂- oder 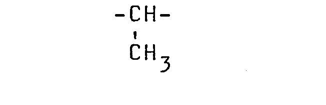 steht, wobei das Sauerstoffatom an den Rest R gebunden ist, indem man
A) Hydrazine der Formel 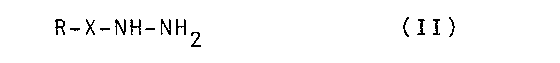 in welcher
R und X die oben angegebene Bedeutung haben, mit ß-Ketosäurederivaten der Formel 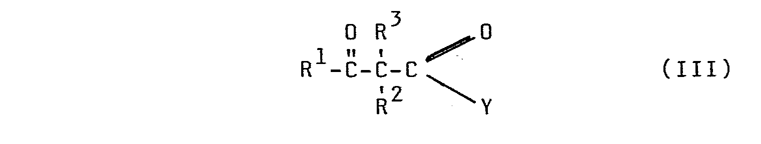 in welcher
_{R}¹, _{R}² und R³ die oben angegebene Bedeutung haben und
Y für einen austretenden Rest, z.B. einen Hydroxy-, Alkoxy-, Aralkoxy-, Amino- oder Alkylaminorest steht,
gegebenenfalls in Gegenwart inerterLösungsmittel und basischer oder saurer Katalysatoren wie Alkali-und Erdalkalihydroxide und -carbonate oder wie Halogenwasserstoffsäuren, Schwefelsäure oder Sulfonsäuren, bei Temperaturen zwischen-10 und 200°C umsetzt,
oder
B) Verbindungen der Formel 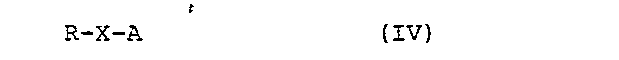 in welcher
R und X die oben angegebene Bedeutung haben und
A einen austretenden Rest wie Halogen oder einen Dialkyloxonium-, Dialkylsulfonium-oder Trialkylammoniumrest oder einen Aryl-oder Trifluormethylsulfonsäurerest darstellt, mit Pyrazolon-(5)-derivaten der Formel 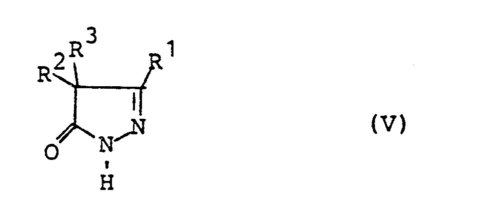 in welcher
R¹, R² und _{R}³ die oben angegebene Bedeutung haben,
gegebenenfalls in Gegenwart inerter Lösungsmittel und anorganischer oder organischer Basen wie Alkalihydroxide, -carbonate, -alkoholate, -hydride oder -amide bei Temperaturen zwischen-10 und 200°C umsetzt.
